# EUROPEAN PATENT APPLICATION

(11) **EP 4 644 555 A1**
(43) Date of publication of application: **05.11.2025**
(21) Application number: 23908881.8
(22) Date of filing: 30.03.2023
(51) Int. Cl.: C12N 15/77, C12N 15/54, C12N 9/12, C12P 13/08, C12N 1/21, C12R 1/15

(54) **GALACTOSE-1-PHOSPHATE URIDYLTRANSFERASE MUTANT AND USE THEREOF IN PREPARATION OF L-LYSINE**

(30) Priority: 29.12.2022 CN 202211706015
(71) Applicant: Ningxia Eppen Biotech Co., Ltd, Yinchuan, Ningxia 750199 (CN)
(72) Inventor: ZHOU, Xiaoqun, Yinchuan, Ningxia 750199 (CN); MENG, Gang, Yinchuan, Ningxia 750199 (CN); WEI, Aiying, Yinchuan, Ningxia 750199 (CN); ZHAO, Chunguang, Yinchuan, Ningxia 750199 (CN); MA, Fengyong, Yinchuan, Ningxia 750199 (CN); ZHANG, Ying, Yinchuan, Ningxia 750199 (CN)
(74) Representative: Isarpatent
(86) International application number: PCT/CN2023/084971
(87) International publication number: WO 2024/138928

(57) **Abstract**

Provided are a galactose-1-phosphate uridyltransferase mutant and the use thereof in the preparation of L-lysine. The galactose-1-phosphate uridyltransferase is A1) or A2): A1) a protein having an amino acid sequence as shown in SEQ ID No. 2; and A2) a fusion protein obtained by linking a tag to the N-terminal or/and the C-terminal of A1). Terminating or knocking out coding genes of the galactose-1-phosphate uridyltransferase in advance can improve the yield of L-lysine in cells.

## Description

### Technical Field

The present invention relates to the field of biotechnology, in particular to a galactose-1-phosphate uridyltransferase mutant and use thereof in the preparation of L-lysine.

### Background Art

L-lysine has physiological effects such as promoting development, enhancing immunity and improving central nervous system functions. It is one of the eight essential amino acids that humans and animals cannot synthesize on their own and are necessary for growth. At present, L-lysine, as the second largest amino acid variety in the world, is mainly produced by a fermentation method, wherein Corynebacterium glutamicum, etc. are important production strains for lysine. L-lysine accounting for approximately 90% of the industrial output is used as a nutritional enhancer in the feed industry, 10% as an umami agent and a sweetener in the food industry, as well as a drug intermediate in the pharmaceutical industry.

At present, L-lysine is mainly produced by a direct fermentation method in which L-lysine is produced by means of aerobic fermentation by using a strain with a complete L-lysine biosynthesis pathway and by taking waste molasses, starch hydrolysate, etc. as substrates. At present, L-lysine fermentation strains used both at home and abroad are mainly mutant strains of Corynebacterium glutamicum, and the main factor affecting their yields mainly focuses on a producing strain, so improving the production ability of L-lysine-producing strains is the focus of current research.

### Summary of the Invention

A technical problem to be solved by the present invention is how to prepare L-lysine.

To solve the above technical problem, the present invention first provides use of a substance for knocking out a protein encoding gene or inhibiting the protein content or activity in preparation of L-lysine;
the protein is A1) or A2) as follows:
A1) a protein having an amino acid sequence of SEQ ID No. 2; and
A2) a fusion protein obtained by linking a tag to an N-terminal or/and a C-terminal of A1).

In the above-mentioned use, the coding gene may be b1) or b2) or b3) as follows:
b1) a DNA molecule as shown in SEQ ID No. 1 in a sequence listing;
b2) a DNA molecule that has 75% or more identity to a nucleotide sequence defined by b1) and encodes the protein; and
b3) a DNA molecule that hybridizes with the nucleotide sequence defined by b1) or b2) under stringent conditions and encodes the protein.

The term "identity" as used herein refers to a sequence similarity to a natural nucleic acid sequence. "Identity" includes a nucleotide sequence that has 75% or more, or 85% or more, or 90% or more, or 95% or more identity to the nucleotide sequence that encodes a protein composed of an amino acid sequence as shown in SEQ ID No. 1 of the present invention. Identity may be evaluated with naked eyes or by computer software. In a case where computer software is utilized, the identity between two or more sequences may be represented by percentage (%) which can be used to evaluate the identity between related sequences.

The stringent conditions may be as follows: hybridizing at 50°C in a mixed solution of 7% sodium dodecyl sulfate (SDS), 0.5 M NaPO₄ and 1 mM EDTA, and rinsing at 50°C in 2×SSC and 0.1% SDS; or hybridizing at 50°C in a mixed solution of 7% SDS, 0.5 M NaPO₄ and 1 mM EDTA, and rinsing at 50°C in 1×SSC and 0.1% SDS; or hybridizing at 50°C in a mixed solution of 7% SDS, 0.5 M NaPO₄ and 1 mM EDTA, and rinsing at 50°C in 0.5×SSC and 0.1% SDS; or hybridizing at 50°C in a mixed solution of 7% SDS, 0.5 M NaPO₄ and 1 mM EDTA, and rinsing at 50°C in 0.1×SSC and 0.1% SDS; or hybridizing at 50°C in a mixed solution of 7% SDS, 0.5 M NaPO₄ and 1 mM EDTA, and rinsing at 65°C in 0.1×SSC and 0.1% SDS; or hybridizing at 65°C in a solution of 6×SSC and 0.5% SDS, and then washing a film once with 2×SSC and 0.1% SDS, and 1×SSC and 0.1% SDS, respectively; or hybridizing at 68°C in a solution of 2×SSC and 0.1% SDS, and washing a film twice at 68°C, 5 min each time, and then hybridizing at 68°C in a solution of 0.5×SSC and 0.1% SDS, and washing a film twice, 15 min each time; or hybridizing in a solution of 0.1×SSPE (or 0.1×SSC) and 0.1% SDS, and washing a film, at 65°C.

The above-mentioned identity of 75% or more may be the identity of 80%, 81%, 82%, 83%, 84%, 85%, 86%, 87%, 88%, 89%, 90%, 91%, 92%, 93%, 94%, 95%, 96%, 97%, 98% or 99%.

In the above-mentioned use, the substance may be B1) or B2):
B1) a nucleic acid molecule that reduces the expression level of the protein; and
B2) an expression cassette, a recombinant vector, a recombinant microorganism or a transgenic cell line containing the nucleic acid molecule of B1).

The nucleic acid molecule of B1) may be DNA, such as cDNA, genomic DNA, or recombinant DNA; and the nucleic acid molecule may also be RNA, such as gRNA, mRNA, siRNA, shRNA, sgRNA, miRNA or antisense RNA.

The expression cassette of B2) refers to DNA capable of expressing the gene in a host cell; and the DNA may not only include a promoter that initiates gene transcription, but also a terminator that terminates the gene transcription. Further, the expression cassette may also include an enhancer sequence.

A recombinant vector containing the gene expression cassette may be constructed using a plant expression vector.

In the above-mentioned use, the substance may be a substance that mutates a glutamate residue codon at position 414 of SEQ ID No. 2 into a terminator; or
the substance may be a substance that mutates a guanine nucleotide at position 1240 of SEQ ID No. 1 into a thymine nucleotide.

The present invention further provides a method for preparing L-lysine. The method includes: reducing the content or activity of the protein in a recipient biological cell, or knocking out an coding gene of the protein in a recipient biological cell to obtain a recombinant biological cell; and culturing the recombinant biological cell to obtain the L-lysine.

The biological cell contains the coding gene of the protein.

As described above, knocking out the coding gene of the protein in the recipient biological cell may be achieved by methods such as gene knockout, gene silencing and the like.

As described above, the gene knockout means that knockout is an exogenous DNA introduction technology in which DNA fragment(s) containing certain known sequences are used to be homologously recombined with gene(s) having the same or similar sequences in a recipient cell genome, and integrated into the recipient cell genome to achieve expression. This technology can change genetic gene(s) of an organism, and enable specific genes to lose their functions, such that some functions are shielded.

As described above, the gene silencing means that gene silencing, also known as gene silence, is a special physiological phenomenon in the regulation process of gene expression of eukaryotic cells, which refers to a "silence" phenomenon occurring in partial segments of a cell gene caused by a combined effect of various factors during the expression of the cell gene, resulting in loss of transcriptional activity thereby no expression or reduced expression.

In the above-mentioned method, the biological cell may be yeast, a bacterium, an algae, a fungus, a plant cell or an animal cell that can synthesize L-lysine.

In the above-mentioned method, the bacterium may be *Corynebacterium glutamicum,* such as *Corynebacterium glutamicum* YP097158.

The bacterium of the present invention includes, but is not limited to, *Corynebacterium glutamicum.* A gene as shown in SEQ ID No. 1 in the sequence listing contained in any bacterium can be mutated or knocked out to produce L-lysine. For example, the bacterium may be *Corynebacterium glutamicum, Escherichia coli, Pantoea ananatis, Bacillus brevis* or *Brevis lactobacillus.*

The above-mentioned method may be achieved by mutating a glutamate residue codon at position 414 of SEQ ID No. 2 in the recipient biological cell into a terminator, or mutating a guanine nucleotide at position 1240 of SEQ ID No. 1 into a thymine nucleotide, or knocking out the gene as shown in SEQ ID No. 1.

In the above-mentioned method, the recombinant biological cell may be cultured using a medium that enables the recombinant biological cell to grow; and/or

the recombinant biological cell may be cultured under conditions that enable the recombinant biological cell to grow.

The recombinant biological cell may be used to produce a variety of products, including but not limited to lysine in the Examples. The resulting products may also be glutamic acid, valine, glycine, alanine, leucine, isoleucine, methionine, proline, tryptophan, serine, tyrosine, cysteine, phenylalanine, asparagine, glutamine, threonine, aspartic acid, arginine, histidine, shikimic acid, protocatechuic acid, succinic acid, α-ketoglutarate, citric acid, ornithine, citrulline, etc.

The present invention further provides a biological material which is b1) or b2) or b3) or b4) or b5) as follows:
b1) a DNA molecule that encodes a protein having an amino acid sequence as shown in SEQ ID No. 6;
b2) a DNA molecule that has 75% or more identity to the sequence of the DNA molecule defined by b1) and encodes a protein as shown in SEQ ID No. 6;
b3) a DNA molecule that hybridizes with a nucleotide sequence defined by b1) or b2) under stringent conditions and encodes a protein as shown in SEQ ID No. 6;
b4) an expression cassette, a recombinant vector, a recombinant microorganism or a transgenic cell line containing the DNA molecule of b1) or b2) or b3); and
b5) the recombinant biological cell.

The cell line may or may not include a propagating material.

The present invention further provides a product for preparing L-lysine, the product containing (or its active ingredient being) the substance or the biological material.

### Description for the deposit of biomaterial

Toxonomic designation: *Corynebacterium glutamicum*
Strain identification reference: YP097158
Depositary authority: China General Microbiological Culture Collection Center
Abbreviation of depositary authority: CGMCC
Address of depositary authority: No. 3, Courtyard 1, Beichen West Road, Chaoyang District, Beijing, Postal Code: 100101
Date of deposit: August 16, 2016
Accession number given by depositary Center: CGMCC No. 12856

### Detailed Description of the Invention

The present invention is described in further detail below in conjunction with the specific embodiments, and the given examples are only for the purpose of clarifying the present invention, but not for the purpose of limiting the scope of the present invention. The examples provided below may be used as a guide for further improvement by a person of ordinary skill in the art and do not in any way constitute a limitation of the present invention.

The experimental methods in the following examples, unless otherwise specified, are conventional methods and are carried out in accordance with the techniques or conditions described in the literatures in the art or in accordance with the product instructions. The materials, reagents, instruments, etc. used in the following examples, unless otherwise specified, may be obtained commercially. In the quantitative test in the following examples, three replicate experiments were set, and the results were averaged. In the following examples, unless otherwise specified, a first position of each nucleotide sequence in the sequence listing was a 5'-end nucleotide of corresponding DNA/RNA, and a last position was a 3'-end nucleotide of the corresponding DNA/RNA.

In the following examples, SPSS11.5 statistical software was used to process data, followed by One-way ANOVA test.

### Example 1: Construction and screening of mutant types of galactose-1-phosphate uridyltransferase NCgl2002 gene beneficial for L-lysine synthesis

### I. Construction of galactose-1-phosphate uridyltransferase NCgl2002 mutant plasmid

First, a wild-type NCgl2002 gene (having a sequence as shown in SEQ ID No. 1) and its promoter sequence were cloned into an expression vector pXMJ19. A wild-type NCgl2002 gene and its promoter sequence (a sequence as shown in SEQ ID No. 3) were obtained by PCR amplification with a *Corynebacterium glutamicum* ATCC13032 genome sequence published by NCBI as a template using primers pXMJ19-PF and pXMJ19-PR, respectively. The recovered product was ligated to an expression vector pXMJ19 (TaKaRa, having chloramphenicol resistance), which was digested by *BamHI*/*EcoRI* enzyme and recovered, through an NEBuilder enzyme (NEB) at 50°C for 30 min; and the ligated product was transformed into DH5α, which was coated onto a 2-YT agar plate containing chloramphenicol (34 mg/L), and cultured at 37°C to obtain a pXMJ19 transformant pXMJ19-NCgl2002 (having a sequence as shown in SEQ ID No. 3) containing the NCgl2002 gene and its promoter sequence. The cultured single clone was subjected to PCR identification with primers M13R(-48)/P1 and r Taq, and a pXMJ19 positive transformant pXMJ19-NCgl2002 containing an NCgl2002 gene and its promoter sequence is the one which was amplified by PCR as having a fragment size of 1465 bp (having a sequence as shown in SEQ ID No. 4) .

In SEQ ID No. 3, positions 42-113 were its promoter sequences.

In order to obtain a mutant that encodes a galactose-1-phosphate uridyltransferase gene NCgl2002, a random mutagenesis kit (Agilent Technologies, USA) was used to prepare an NCgl2002 mutant gene plasmid. Using the plasmid pXMJ19-NCgl2002 as a template, PCR amplification was performed respectively using primers pXMJ19-PF/pXMJ19-PR to obtain an NCgl2002 gene fragment (1441 bp) containing random point mutations, a pXMJ19-NCgl2002 positive transformant containing the NCgl2002 gene,i.e., pXMJ19-NCgl2002-MT (having a sequence as shown in SEQ ID No. 3, with random point mutations occurring in an NCgl2002 coding region). The recovered DNA fragment was ligated to an expression vector pXMJ19 (TaKaRa, having chloramphenicol resistance), which was digested by *BamH*I/*EcoR*I enzyme and recovered, through an NEBuilder enzyme (NEB) at 50°C for 30 min; and the ligated product was transformed into DH5α, which was coated onto a 2-YT agar plate containing chloramphenicol (34 mg/L), and cultured at 37°C. The cultured single clone was subjected to PCR identification with primers M13R(-48)/P1 and r Taq, and a pXMJ19 positive transformant containing an NCgl2002 random mutation is the one which was amplified by PCR as having a fragment size of 1465 bp (having a sequence as shown in SEQ ID No. 4, with random point mutations occurring in an NCgl2002 coding region).

The primer design was as follows (synthesized by Shanghai Invitrogen Company):
pXMJ19-PF:
   5'-AATTAAGCTTGCATGCCTGCAGGTCGACTCTAGAGGATCCCaacaccacagtagacaatagccttg-3' (the underlined nucleotide sequence was a pXMJ19 homologous arm sequence) (SEQ ID No. 9); and
pXMJ19-PR:
   5'-GAAAATCTTCTCTCATCCGCCAAAACAGCCAAGCTGAATTCttataggaggggattgtatttaagg-3 ' (the underlined nucleotide sequence was a pXMJ19 homologous arm sequence) (SEQ ID No. 10).
   M13R(-48): 5'-AGCGGATAACAATTTCACACAGGA -3' (SEQ ID No. 11); and
   P1: 5'-CTCTCATCCGCCAAAACAG -3' (SEQ ID No. 12).

### II. Screening of mutant types of galactose-1-phosphate uridyltransferase NCgl2002 gene beneficial for L-lysine synthesis

In order to identify the L-lysine production properties of the mutant vector constructed in Step I, the NCgl2002 random mutation plasmids constructed in Step I were respectively electroprorated into Corynebacterium glutamicum YP097158 (Accession number: CGMCC No. 12856; date of deposit: August 16, 2016; depositary authority: the Institute of Microbiology at the Chinese Academy of Sciences, No. 3, Courtyard 1, Beichen West Road, Chaoyang District, Beijing; Telephone number: 010-64807355), and cultured in a medium containing chloramphenicol (34 mg/L). The medium composition and culture conditions were shown in Table 1. The cultured monoclone was subjected to PCR identification with primers M13R(-48)/P1 and r Taq, and a pXMJ19 positive transformant containing an NCgl2002 random mutation is the one which was amplified by PCR as having a fragment size of 1465 bp (having a sequence as shown in SEQ ID No. 4, with random point mutations occurring in an NCgl2002 coding region).

The positive transformant was cultured in a medium containing chloramphenicol (34 mg/L). The medium composition and culture conditions were shown in Table 1. After three consecutive passages, it was inoculated into a 500 mL triangular flask filled with 30 mL of rich medium and fermented at 37°C for 24 h under shaking. When fermentation culture bacteria grew to OD₆₀₀=0.1, IPTG at a final concentration of 0.1 mM was added to induce overexpression of an NCgl2002 protein.

After the fermentation culture, the concentration of L-amino acid was analyzed by high performance liquid chromatography (HPLC), as shown in Table 2. Strains (i.e., YP097158-pXMJ19-NCgl2002 mutant strains) with superior L-amino acid production capacity to a Corynebacterium glutamicum YP097158 control were selected.

Rich medium: water was used as a solvent; solutes and concentration thereof were 30 g/L glucose, 2 g/L (NH4)₂SO₄, 0.5 g/L H₃PO₄, 0.8 g/L KCl, 0.8 g/L MgSO₄ . 7H₂O, 0.05 g/L FeSO₄ . 7H₂O, 0.05 g/L MnSO₄ . H₂O, 1.5 g/L FM902 yeast powder, 1.5 g/L corn syrup, 17 g/L molasses, 0.5 g/L betaine, 2 g/L citric acid, 20 mg/L VH, 1.5 mg/L VB₁, 1.5 mg/L VB₃, 1.5 g/L VB₁₂, and pH was adjusted to 7.0 with sodium hydroxide.

**Table 1. Compositions and culture conditions of media**

| | Components | Content |
|---|---|---|
| Media | Sucrose | 10 g/L |
| | Polypeptone | 10 g/L |
| | Beef extract | 10 g/L |
| | Yeast powder | 5 g/L |
| | Urea | 2 g/L |
| | Sodium chloride | 2.5 g/L |
| | Agar powder | 20 g/L |
| | Water | |
| | pH7.0 | |
| Culture conditions | Culture temperature: 32°C | |
| | Culture time: 40 h | |

**Table 2: Analysis results of L-amino acids of YP097158-NCgl2002 mutant strains by high-performance liquid chromatography**

| Names of L-amino acids | Content of L-amino acids (g/100mL) | | | | | |
|---|---|---|---|---|---|---|
| | YP09 7158 | YP097158-N Cgl2002 mutant strain 1 | YP097158-N Cgl2002 mutant strain 2 | YP097158-N Cgl2002 mutant strain 3 | YP097158-N Cgl2002 mutant strain 4 | YP097158-N Cgl2002 mutant strain 5 |
| L-asparti c acid | Not detect ed | Not detected | Not detected | Not detected | Not detected | Not detected |
| L-glutami c acid | 0.015 | 0.027 | 0.017 | 0.023 | 0.013 | 0.031 |
| L-serine | Not detect ed | Not detected | Not detected | Not detected | Not detected | Not detected |
| L-arginin e | 0.004 | 0.001 | 0.001 | 0.007 | 0.014 | 0.021 |
| L-glycine | Not detect ed | Not detected | Not detected | Not detected | Not detected | Not detected |
| L-threoni ne | 0.004 | Not detected | 0.001 | Not detected | 0.001 | Not detected |
| L-lysine | 19.3 | 18.2 | 18.5 | 19.2 | 18.1 | 18.3 |
| L-proline | Not detect ed | Not detected | Not detected | Not detected | Not detected | Not detected |
| L-alanine | Not detect ed | Not detected | Not detected | Not detected | Not detected | Not detected |
| L-valine | 0.002 | 0.005 | Not detected | 0.002 | Not detected | Not detected |
| L-methio nine | 0.001 | Not detected | Not detected | Not detected | Not detected | Not detected |
| L-cystein e | Not detect ed | Not detected | Not detected | Not detected | Not detected | Not detected |
| L-isoleuc ine | 0.001 | Not detected | 0.001 | Not detected | Not detected | Not detected |
| L-leucine | Not detect ed | Not detected | Not detected | Not detected | Not detected | Not detected |
| L-phenyl alanine | Not detect ed | Not detected | Not detected | Not detected | Not detected | Not detected |
| L-tyrosin e | Not detect ed | Not detected | Not detected | Not detected | Not detected | Not detected |

As shown in Table 2, among *Corynebacterium glutamicum* YP097158-NCgl2002 mutant strains, the YP097158-NCgl2002 mutant strain 3 could maintain the production ability of L-lysine, and the rest could reduce the production ability of L-lysine. It showed that this gene played a role in inhibiting L-lysine production, and the NCgl2002 mutant strain 3 could inactivate this gene, thereby maintaining the ability to synthesize L-lysine.

It was confirmed through the results of sequencing the NCgl2002 gene by extracting a plasmid from the Corynebacterium glutamicum YP097158 mutant strain 3 that, an NCgl2002 mutation site was a mutation from guanine (G) at position 1240 in an coding region of this gene to thymine (T) (a gene containing this mutation was denoted as an NCgl2002^{G1240T} gene); and glutamic acid (E) at position 414 in an amino acid sequence of its mutant protein NCgl2002 was mutated to a terminator (*) (a protein containing this mutation was denoted as an NCgl2002^{Gl240T} protein); and this plasmid was pXMJ19-E414* (having a sequence as shown in SEQ ID No. 3, and position 1353 of this sequence was mutated to T). Wherein, the DNA sequence as shown in SEQ ID No. 1 was a wild-type NCgl2002 gene, with an amino acid sequence of an encoding protein being SEQ ID No. 2 (this protein was named as a wild-type NCgl2002 protein).Wherein, the DNA sequence as shown in SEQ ID No. 5 was a mutant NCgl2002^{G1240T} gene, thymine (T) at position 1240 in the mutant NCgl2002^{G1240T} gene sequence (SEQ ID No. 5) was mutated from guanine (G), with an amino acid sequence of the encoding protein being SEQ ID No. 6 (the mutant protein was named as a mutant NCgl2002^{E414*} protein), a terminator (*) at position 414 in an amino acid sequence (SEQ ID No. 6) of the mutant protein NCgl2002^{E414*} was mutated from glutamate (E).

### Example 2: Construction of mutant engineered strain containing NCgl2002 gene in genome

Based on a genome sequence of Corynebacterium glutamicum YP097158 or wild-type Corynebacterium glutamicum strain ATCC13032, a more in-depth study on the effects of the NCgl2002 gene and the mutant NCgl2002^{E414*} gene on the yield of L-lysine was carried out in high-yield strains by using an allelic substitution method.

A point mutation was introduced into a coding region (SEQ ID No. 1) of the NCgl2002 gene. The point mutation was to mutate guanine (G) at position 1240 in the nucleotide sequence (SEQ ID No. 1) of the NCgl2002 gene into thymine (T) to obtain the DNA molecule (the mutant NCgl2002 gene, named as the mutant NCgl2002^{E414*} gene) as shown in SEQ ID No. 5.

An amino acid sequence of the protein encoded by the DNA molecule as shown in SEQ ID No. 1 was SEQ ID No. 2 (the name of the protein was the wild-type NCgl2002 protein). The amino acid sequence of the protein encoded by the DNA molecule as shown in SEQ ID No. 5 was SEQ ID No. 6 (the name of the mutant protein was the mutant NCgl2002^{E414*} protein), and glutamic acid (E) at position 414 in the amino acid sequence (SEQ ID No. 6) of the mutant protein NCgl2002^{E414*} was mutated to a terminator (*).

### I. Construction of recombinant vector with coding region of mutant NCgl2002^{E414*} gene

With Corynebacterium glutamicum YP097158 or wild-type Corynebacterium glutamicum strain ATCC13032 genome DNA as a template, two NCgl2002^{E414*} DNA fragments (NCgl2002^{E414*}Up and NCgl2002^{E414*}Down) with mutant base sizes of 525 bp and 533 bp respectively were obtained by performing PCR amplification with primers P2/P3, P4/P5 and KAPA HiFi HotStart, respectively. After the PCR reaction, agarose gel electrophoresis was performed by a column DNA gel recovery kit to recover NCgl2002^{E414*}Up and NCgl2002^{E414*}Down, respectively. The recovered DNA was subjected to overlap PCR by primers P2/P5 to obtain a DNA fragment Up-NCgl2002^{E411*}-Down (SEQ ID No. 7) (1022 bp) of a point mutation-integrated homologous arm.

The primer design was as follows (synthesized by Shanghai Invitrogen Company):
P2: 5'-**CAGTGCCAAGCTTGCATGCCTGCAGGTCGACTCTAG**GAACTGCATCATCTACGT GG-3', (the underlined nucleotide sequence was a sequence on pK18) (SEQ ID No. 13),
P3: 5'-GCGTTCAACG GAGCATTACA TGGCGATGCG-3' (SEQ ID No. 14),
P4: 5'-GATCTGCGCATCGCCATGTAATGCTCCGTTG-3' (SEQ ID No. 15),
P5: 5'-**CAGCTATGACCATGATTACGAATTCGAGCTCGGTACCC**GCACCAAGCAGCGCGG TGAC-3', (the underlined nucleotide sequence was a sequence on pK18) (SEQ ID No. 16).

The DNA fragment (Up-NCgl2002^{E414*}-Down) of the above-mentioned point mutation-integrated homologous arm obtained by overlap PCR was separated and purified by agarose gel electrophoresis, and then linked to a pK18mobsacB plasmid (Addgene) purified by enzyme digestion (*Xbal* I and *BamH* I) through an NEBuilder enzyme (NEB) at 50°C for 30 min. After the linking product was transformed into Escherichia coli DH5a and grew into a monoclonal, the monoclonal was subjected to PCR identification through primers M13F/M13R (M13F: 5'-TGTAAAACGACGGCCAGT-3' (SEQ ID No. 17), M13R: 5'-CAGGAAACAGCTATGACC-3' (SEQ ID No. 18)). A plasmid was extracted to obtain a positive recombinant vector with a correct sequence, which was denoted as pK18-NCgl2002^{G1240T}. This recombinant vector contained a kanamycin resistance marker.

The NCgl2002^{G1240T}Up-Down DNA in this recombinant vector pK18-NCgl2002G^{1240T} had a size of 1022 bp (SEQ ID No. 7) and contained a mutation site (G-T), such that guanine (G) at position 1240 in coding regions of the NCgl2002 gene in Corynebacterium glutamate YP097158 and wild-type Corynebacterium glutamate ATCC13032 into thymine (T), eventually causing glutamic acid (E) at position 414 in the encoding protein to be mutated into a terminator (*).

The recombinant vector pK18-NCgl2002^{G1240T} was a recombinant vector which was obtained by replacing fragments (small fragments) between *Xbal* I and *BamH* I recognition sites of a pK18mobsacB vector with the DNA fragment as shown in SEQ ID No. 7 in the sequence listing, and keeping the other sequences of the pK18mobsacB vector unchanged. The recombinant vector pK18-NCgl2002^{G1240T} contained a mutation site (G-T) of the mutant gene NCgl2002^{G1240T} as shown in SEQ ID No. 5.

### II: Construction of engineered strain containing NCgl2002^{G1240T} in genome

The above allelic substitution plasmid (pK18-NCgl2002^{G1240T}) was transformed by electric shock into L-lysine-producing bacteria (*Corynebacterium glutamicum* YP097158) and a wild-type Corynebacterium glutamicum strain ATCC13032 (transformation method was the same as above), and cultured on a solid culture plate containing kanamycin (see Table 1 for the medium composition and culture conditions). Single colonies produced in culture were identified by the above-mentioned primer P2 and the general primer M13R, respectively. A strain that can amplify a stripe of 1070 bp was a positive strain. The positive strain was cultured on a medium containing 15% of sucrose (the medium was obtained by increasing the sucrose concentration in the medium in Table 1 to 15 g/L). The single colonies produced in culture were cultured on a kanamycin-containing medium and a kanamycin-free medium, respectively. Strains that grew on the kanamycin-free medium, but did not grow on the kanamycin-containing medium were further subjected to PCR amplification using the following primers (synthesized by Shanghai invitrogen):
P6: 5'-CTACCCTGGCAGGTTTTGAAG-3' (SEQ ID No.19);
P7: 5'- GAAGTTCTGA AATGCGGCTC-3' (SEQ ID No.20).

The obtained DNA fragment (256 bp) was treated (denaturated at a high temperature of 95°C for 10 min, and quickly placed in an ice bath for 5 min) and then subjected to single-strand conformation polymorphis (SSCP) electrophoresis (an amplified fragment of the plasmid pK18-NCgl2002^{G1240T} was used as a positive control, an amplified fragment of Corynebacterium glutamate ATCC13032 was used as a negative control, and water was used as a blank control). The preparation and electrophoresis conditions of PAGE for SSCP electrophoresis were shown in Table 3. Due to different fragment structures and different electrophoresis positions, the strains whose fragment electrophoresis positions were inconsistent with segment positions of the negative control and but consistent with fragment positions of the positive control were strains undergoing successful allelic substitution. A positive strain NCgl2002^{Gl240T} gene fragment was then subjected to PCR amplification through primers P6/P7 and linked to a PMD19-T vector for sequencing. Through sequence alignment, the strains with whose base sequences having mutations (G-T) were strains undergoing successful allelic substitution. Positive strains obtained by Corynebacterium glutamicum YP097158 and wild-type Corynebacterium glutamicum strain ATCC13032 were named as YPL-NCgl2002-1, and L2002-1.

Both the recombinant bacteria YPL-NCgl2002-1 and L2002-1 contained a mutated gene NCgl2002^{G1240T} as shown in SEQ ID No. 5 and could express the protein as shown in SEQ ID No. 6. The only difference between the recombinant bacteria YPL-NCgl2002-1 and *Corynebacterium glutamicum* YP097158 resided in that: YPL-NCgl2002-1 was a strain which was obtained by replacing an NCgl2002 gene of *Corynebacterium glutamicum* YP097158 with an NCgl2002^{Gl240T} gene and keeping other sequences unchanged. The only difference between recombinant bacteria L2002-1 and ATCC13032 resided in that L2002-1 was a strain which was obtained by replacing an NCgl2002 gene of ATCC13032 with an NCgl2002^{G1240T} gene and keeping other sequences unchanged.

**Table 3: Preparation and electrophoresis conditions of PAGE for SSCP electrophoresis**

| | Components | Dosage (final acrylamide concentration of 8%) |
|---|---|---|
| PAGE | 40% acrylamide | 8 mL |
| | ddH₂O | 26 mL |
| | Glycerinum | 4 mL |
| | 10×TBE | 2 mL |
| | TEMED | 40 µL |
| | 10%APS | 600 µL |
| Electrophoresis conditions | An electrophoresis tank was placed into ice, 1×TBE buffer was used, the voltage was 120 V, and the electrophoresis time was 10 h. | |

### Example 3: Construction of engineered strain with NCgl2002 gene deficient in genome

According to a genomic sequence of *Corynebacterium glutamicum* ATCC13032 published by NCBI, two pairs of primers that amplify the fragments at both ends of an coding region of the NCgl2002 gene were synthesized as upstream and downstream homologous arm fragments. The primer design was as follows (synthesized by Shanghai Invitrogen Company):
P8: 5' -CAGTGCCAAGCTTGCATGCCTGCAGGTCGACTCTAGCGTGATGCAGGCCGAAGGATC-3' (the underlined nucleotide sequence was a sequence on pK18) (SEQ ID No. 21),
P9: 5'- GCGACACTAAAACTCTTGGCGGTGCGAATGGGGGTGACAG-3' (SEQ ID No. 22),
P10: 5'- CTGTCACCCCCATTCGCACCGCCAAGAGTTTTAGTGTCGC-3' (SEQ ID No. 23),
P11: 5' -CAGCTATGACCATGATTACGAATTCGAGCTCGGTACCCGGAGTTTTCCTCCGATGGCTG -3' (the underlined nucleotide sequence was a sequence on pK18) (SEQ ID No. 24).

Construction method: with Corynebacterium glutamicum ATCC13032 as a template, PCR amplification was performed using primers P8/P9 and P10/P11 respectively to obtain an upstream homologous arm fragment (571 bp) and a downstream homologous arm fragment (566 bp) from which NCgl2002 was knocked out. The amplified product was subjected to electrophoresis and purified by a column DNA gel recovery kit. The recovered DNA fragments were linked to a pK18mobsacB plasmid (Addgene) purified by *Xbal* I/*BamH I* enzyme digestion through an NEBuilder enzyme (NEB) at 50°C for 30 min. A monoclonal grown after transformation of the linking product was subjected to PCR identification using an M13 primer to obtain a positive knockout vector pK18-ΔNCgl2002. This plasmid contained the entire homologous arm fragment (1097 bp) (having a sequence as shown in SEQ ID No. 8) from which NCgl2002 was knocked out and had kanamycin resistance as a screening marker. This plasmid was delivered for sequencing. The correctly sequenced knockout plasmid pK18-ΔNCgl2002 was electrotransformed into Corynebacterium glutamate YP097158 and wild-type Corynebacterium glutamate ATCC13032, and cultured in a medium. The medium composition and culture conditions were shown in Table 1. Single colonies produced in culture were subjected to PCR identification through primers P8/P11: the strains that amplified the stripes of 1097 bp and 2384 bp at the same time were both positive strains, and the strains that amplified a stripe of 2384 bp only were probiotics. The positive strains were screened on a 15% sucrose solid medium, and then cultured on a kanamycin-containing medium and a kanamycin-free medium, respectively. The strains that grew on the kanamycin-free medium, but did not grow on the kanamycin-containing medium were selected, and further subjected to PCR identification through primers P8/P11. The strains that amplified a stripe of 1097 bp were positive strains whose coding region of the NCgl2002 gene was knocked out. The positive strain NCgl2002 fragment was subjected to PCR amplification again through primers P8/P11 and linked to a pMD19-T vector for sequencing. The correctly sequenced strains were named as YPL-NCgl2002-2 (the NCgl2002 gene on the YP097158 genome of Corynebacterium glutamate was knocked out) and L2002-2 (the NCgl2002 gene on the wild-type Corynebacterium glutamate ATCC13032 genome was knocked out).

### Example 4: L-lysine fermentation experiment

A fermentation experiment was performed on the strains constructed in Examples 2 and 3 and original strains YP097158 and ATCC13032 of Corynebacterium glutamicum in a fermentation tank of BLBIO-5GC-4-H model (Shanghai Bailun Biotechnology Co., Ltd.) using media shown in Table 4 and the control process shown in Table 5. After the fermentation, the L-lysine yield was detected by ninhydrin colorimetry. Each strain was repeated three times, and the results were shown in Table 6.

**Table 4: Formula of fermentation medium**

| Components | Formula |
|---|---|
| Starch hydrolyzed sugar | 30 g/L |
| Ammonium sulfate | 12 g/L |
| Magnesium sulfate | 0.87 g/L |
| Molasses | 20 g/L |
| Acidified corn syrup | 3 mL/L |
| Phosphoric acid | 0.4 mL/L |
| Potassium chloride | 0.53 g/L |
| Defoamer (2% Dipao) | 4 mL/L |
| Ferrous sulfate | 120 mg/L |
| Manganese sulfate | 120 mg/L |
| Nicotinamide | 42 mg/L |
| Calcium pantothenate | 6.3 mg/L |
| Vitamin B1 | 6.3 mg/L |
| Copper and zinc salt solution | 0.6 g/L |
| Biotin | 0.88 mg/L |

**Table 5: Fermentation control process**

| | | | |
|---|---|---|---|
| Corrected to DO100% | Temperature of 37°C, air volume of 4 L/min, speed of 1000 rpm, tank pressure of 0 Mpa, and calibrated after 5 min | | |
| Inoculation amount | 10% | Culture temperature °C | 37°C |
| pH | pH6.9±0.05 | Dissolved oxygen DO | 10-30% |
| Initial conditions | Temperature of 37°C, pH of 6.9, tank pressure of 0 Mpa, air volume of 3 L/min, and speed of 550 rpm | | |
| Whole-process control | Whole-process control 1, the dissolved oxygen < 30%, and the speed was sequentially increased by 750 rpm→800 rpm- air volume of 4 L/min→850 rpm→950 rpm; 2, the tank pressure was increased by 0.01 Mpa after 6 h of fermentation, and the tank pressure was increased by 0.02 Mpa→0.03 Mpa→ 0.04 Mpa-0.05 Mpa after 12 h of fermentation | | |
| Residual sugar control | 0.1-0.2% before F12h; 0.1-0.05% after F12h in combination with DO requirements | | |
| Ammonia-nitrog en control | 0.1-0.15 before F12h; 0.15-0.25 for F12-F32h; 0.1-0.15 after F32h | | |
| Material fed-batch: | 25% of ammonia, 70% of concentrated sugar, 50% of ammonium sulfate, and 10% of Paodi | | |
| Fermentation cycle | About 48 h | | |

**Table 6: yield of L-lysine in NCgl2002 engineered strains**

| Strains | L-lysine concentration (g/100ml) | | | Mean | P value |
|---|---|---|---|---|---|
| YP097158 | 19.2 | 18.9 | 19.3 | 19.133 | |
| YPL-NCgl2002-1 | 19.8 | 19.5 | 19.6 | 19.633 | P<0.05 |
| YPL-NCgl2002-2 | 19.7 | 19.9 | 19.6 | 19.733 | P<0.05 |
| ATCC13032 | 0.3 | 0.5 | 0.2 | 0.333 | |
| L2002-1 | 0.6 | 0.8 | 0.7 | 0.700 | P<0.05 |
| L2002-2 | 0.8 | 1.1 | 0.7 | 0.867 | P<0.05 |

The results were shown in Table 6. Point mutation of NCgl2002^{Gl240T} and knockout of the coding region of the NCgl2002 gene in Corynebacterium glutamicum contributed to the increase in yield and growth rate of L-lysine.

The present invention is detailed above. For a person skilled in the art, the present invention may be implemented within a wide range under the same parameters, concentrations and conditions without departing from the purpose and scope of the present invention and without unnecessary experiments. Although special examples are given in the present invention, it should be understood that further improvements may be made to the present invention. In summary, according to the principles of the present invention, the present application is intended to include any change, use or improvement of the present invention, including changes that deviate from the scope disclosed in the present application and are made by conventional technologies known in the art. According to the scope of the claims attached below, some basic features can be applied. Sequences 1-8 involved in the above examples were as follows:
SEQ ID No. 1: wild-type ORF (CDS) sequence (nucleotide sequence of 1287 bp) of NCgl2002 gene 
ATGGCTGACGGCACGATCAAACAGATTCACCCTTTCACAGGCACCGAAGTGTGGACG
GTCCCTGGGCGTGGAAATCGACCTCTGTCACATCCCGCTTCTACGATCGTCGAACTATC
TGCACACGATCACACCTCTTACTGTGCATTTTGTTCCGACAATATGCTCTCCACTCCGCC
TGAGAAATCGCGCATCATCATTGATAGCTCCGGCGACTTTGACATCCTTCCCGGAGCAT
TGCCTGGTGAGCTTTCAGAAACCACTCCGGAATTTCGACGAGTCCCCAATCTGTTTGA
GATTGTCTCTTTTGACTACTGGCACCAGAATTTTGGTTTCGATATGGATTCAGAAACCG
CCATGCGCATGGCGCAATACTTGGCGATTCCAGAAGGTCGCGAACATGTTTTAGCCATT
GTGCGCACCCGACTTTCTGCCGCTGGTGAAGATCCCGCGCACATGACCGATGGCGAGT
TGTTAGAAAAAGCTCCCAGCTACTTTGCTGGTGGTCATGACGTCATCATCGGACGCCG
ACACTTTGTCGATGACGCAACCACCAGTGATCAATTGGCCTCATCTGGAACACTGACC
GTTAAAGAGCATGAGGCGTTCATCCGCCTGACTGTCGATGGCATCAGGGATTTGTACCA
CCGCAACCGTTACGCACCGTATGTAGTGGCGTTTCAAAACTGGTTGAAACCCGCCGGC
GCGTCTTTTGACCATCTTCATAAACAGCTCGTCGCCATTGATGAACGCGGCCGACTTAT
TGCCGATGAACTGCATCATCTACGTGGCAATCCCAATATGTACAACGAACTTGCTGTTG
ATTACGCCGGATACCACAACCTGATCATCGCGGAAAACGATCACGCCGTGGCCTTCGC
AGGTTTCGGTCACCGCTACCCCACCATTGAGATTTACTCTAAGTCCGCTATTCCTGAAC
CCTGGCTTCAAAGCGACGAGGAAATCCAAGCGATGAGCAACCTCATCCATGCATGCCA
TGCTGCAACCGGCGCAGATGTACCCTGCAATGAGGAATGGGTACACAAACCAATCGAT
GTTGATATGCCAATGCCCTGGCATGTGATGATCAAATGGCGTGTTTCTACCCTGGCAGG
TTTTGAAGGTGGCACCAAGGTGTATCTCAATACGCTGTCTCCACACAAGGTCCGAGAC
CGTGTGGTGAAAGAAATGTACCGACTACGCGATGAAGAACTCATCGCATCTGATCTGC
GCATCGCCATGGAATGCTCCGTTGAACGCAACAGCCTTAAATACAATCCCCTCCTATAA
SEQ ID No. 2: NCgl2002 protein sequence (i.e., amino acid sequence 428aa encoded by Sequence 1) 
MADGTIKQIHPFTGTEVWTVPGRGNRPLSHPASTIVELSAHDHTSYCAFCSDNMLSTPPEK
SRIIIDSSGDFDILPGALPGELSETTPEFRRVPNLFEIVSFDYWHQNFGFDMDSETAMRMAQ
YLAIPEGREHVLAIVRTRLSAAGEDPAHMTDGELLEKAPSYFAGGHDVIIGRRHFVDDATT
SDQLASSGTLTVKEHEAFIRLTVDGIRDLYHRNRYAPYVVAFQNWLKPAGASFDHLHKQL
VAIDERGRLIADELHHLRGNPNMYNELAVDYAGYHNLIIAENDHAVAFAGFGHRYPTIEIY
 
SKSAIPEPWLQSDEEIQAMSNLIHACHAATGADVPCNEEWVHKPIDVDMPMPWHVMIKW
RVSTLAGFEGGTKVYLNTLSPHKVRDRVVKEMYRLRDEELIASDLRIAMECSVERNSLKY
NPLL
**SEQ ID No. 3: pXMJ19-NCgl2002 sequence (nucleotide sequence of 1441 bp) of pXMJ19-integrated NCgl2002 gene and its promoter** 
AATTAAGCTTGCATGCCTGCAGGTCGACTCTAGAGGATCCCAACACCACAGTAGACAA
TAGCCTTGGTGTTATGACTAGCCCCCATTCTTTTTCTGTCACCCCCATTCGCACCATGGC
TGACGGCACGATCAAACAGATTCACCCTTTCACAGGCACCGAAGTGTGGACGGTCCCT
GGGCGTGGAAATCGACCTCTGTCACATCCCGCTTCTACGATCGTCGAACTATCTGCACA
CGATCACACCTCTTACTGTGCATTTTGTTCCGACAATATGCTCTCCACTCCGCCTGAGA
AATCGCGCATCATCATTGATAGCTCCGGCGACTTTGACATCCTTCCCGGAGCATTGCCT
GGTGAGCTTTCAGAAACCACTCCGGAATTTCGACGAGTCCCCAATCTGTTTGAGATTG
TCTCTTTTGACTACTGGCACCAGAATTTTGGTTTCGATATGGATTCAGAAACCGCCATG
CGCATGGCGCAATACTTGGCGATTCCAGAAGGTCGCGAACATGTTTTAGCCATTGTGCG
CACCCGACTTTCTGCCGCTGGTGAAGATCCCGCGCACATGACCGATGGCGAGTTGTTA
GAAAAAGCTCCCAGCTACTTTGCTGGTGGTCATGACGTCATCATCGGACGCCGACACT
TTGTCGATGACGCAACCACCAGTGATCAATTGGCCTCATCTGGAACACTGACCGTTAA
AGAGCATGAGGCGTTCATCCGCCTGACTGTCGATGGCATCAGGGATTTGTACCACCGC
AACCGTTACGCACCGTATGTAGTGGCGTTTCAAAACTGGTTGAAACCCGCCGGCGCGT
CTTTTGACCATCTTCATAAACAGCTCGTCGCCATTGATGAACGCGGCCGACTTATTGCC
GATGAACTGCATCATCTACGTGGCAATCCCAATATGTACAACGAACTTGCTGTTGATTA
CGCCGGATACCACAACCTGATCATCGCGGAAAACGATCACGCCGTGGCCTTCGCAGGT
TTCGGTCACCGCTACCCCACCATTGAGATTTACTCTAAGTCCGCTATTCCTGAACCCTG
GCTTCAAAGCGACGAGGAAATCCAAGCGATGAGCAACCTCATCCATGCATGCCATGCT
GCAACCGGCGCAGATGTACCCTGCAATGAGGAATGGGTACACAAACCAATCGATGTTG
ATATGCCAATGCCCTGGCATGTGATGATCAAATGGCGTGTTTCTACCCTGGCAGGTTTT
GAAGGTGGCACCAAGGTGTATCTCAATACGCTGTCTCCACACAAGGTCCGAGACCGTG
TGGTGAAAGAAATGTACCGACTACGCGATGAAGAACTCATCGCATCTGATCTGCGCAT
CGCCATGGAATGCTCCGTTGAACGCAACAGCCTTAAATACAATCCCCTCCTATAAGAAT
TCAGCTTGGCTGTTTTGGCGGATGAGAGAAGATTTTC
**SEQ ID No. 4: amplified fragment (nucleotide sequence of 1465 bp) of identification primers M13R(-48)/P1** 
AGCGGATAACAATTTCACACAGGAAACAGAATTAATTAAGCTTGCATGCCTGCAGGTC
GACTCTAGAGGATCCCAACACCACAGTAGACAATAGCCTTGGTGTTATGACTAGCCCCC
ATTCTTTTTCTGTCACCCCCATTCGCACCATGGCTGACGGCACGATCAAACAGATTCAC
 
CCTTTCACAGGCACCGAAGTGTGGACGGTCCCTGGGCGTGGAAATCGACCTCTGTCAC
ATCCCGCTTCTACGATCGTCGAACTATCTGCACACGATCACACCTCTTACTGTGCATTTT
GTTCCGACAATATGCTCTCCACTCCGCCTGAGAAATCGCGCATCATCATTGATAGCTCC
GGCGACTTTGACATCCTTCCCGGAGCATTGCCTGGTGAGCTTTCAGAAACCACTCCGG
AATTTCGACGAGTCCCCAATCTGTTTGAGATTGTCTCTTTTGACTACTGGCACCAGAAT
TTTGGTTTCGATATGGATTCAGAAACCGCCATGCGCATGGCGCAATACTTGGCGATTCC
AGAAGGTCGCGAACATGTTTTAGCCATTGTGCGCACCCGACTTTCTGCCGCTGGTGAA
GATCCCGCGCACATGACCGATGGCGAGTTGTTAGAAAAAGCTCCCAGCTACTTTGCTG
GTGGTCATGACGTCATCATCGGACGCCGACACTTTGTCGATGACGCAACCACCAGTGA
TCAATTGGCCTCATCTGGAACACTGACCGTTAAAGAGCATGAGGCGTTCATCCGCCTG
ACTGTCGATGGCATCAGGGATTTGTACCACCGCAACCGTTACGCACCGTATGTAGTGGC
GTTTCAAAACTGGTTGAAACCCGCCGGCGCGTCTTTTGACCATCTTCATAAACAGCTC
GTCGCCATTGATGAACGCGGCCGACTTATTGCCGATGAACTGCATCATCTACGTGGCAA
TCCCAATATGTACAACGAACTTGCTGTTGATTACGCCGGATACCACAACCTGATCATCG
CGGAAAACGATCACGCCGTGGCCTTCGCAGGTTTCGGTCACCGCTACCCCACCATTGA
GATTTACTCTAAGTCCGCTATTCCTGAACCCTGGCTTCAAAGCGACGAGGAAATCCAA
GCGATGAGCAACCTCATCCATGCATGCCATGCTGCAACCGGCGCAGATGTACCCTGCAA
TGAGGAATGGGTACACAAACCAATCGATGTTGATATGCCAATGCCCTGGCATGTGATGA
TCAAATGGCGTGTTTCTACCCTGGCAGGTTTTGAAGGTGGCACCAAGGTGTATCTCAAT
ACGCTGTCTCCACACAAGGTCCGAGACCGTGTGGTGAAAGAAATGTACCGACTACGC
GATGAAGAACTCATCGCATCTGATCTGCGCATCGCCATGGAATGCTCCGTTGAACGCAA
CAGCCTTAAATACAATCCCCTCCTATAAGAATTCAGCTTGGCTGTTTTGGCGGATGAGA
G
**SEQ ID No. 5: ORF (CDS) sequence (nucleotide sequence of 1287 bp) of gene mutant NCgl2002^{G1240T}** 
ATGGCTGACGGCACGATCAAACAGATTCACCCTTTCACAGGCACCGAAGTGTGGACG
GTCCCTGGGCGTGGAAATCGACCTCTGTCACATCCCGCTTCTACGATCGTCGAACTATC
TGCACACGATCACACCTCTTACTGTGCATTTTGTTCCGACAATATGCTCTCCACTCCGCC
TGAGAAATCGCGCATCATCATTGATAGCTCCGGCGACTTTGACATCCTTCCCGGAGCAT
TGCCTGGTGAGCTTTCAGAAACCACTCCGGAATTTCGACGAGTCCCCAATCTGTTTGA
GATTGTCTCTTTTGACTACTGGCACCAGAATTTTGGTTTCGATATGGATTCAGAAACCG
CCATGCGCATGGCGCAATACTTGGCGATTCCAGAAGGTCGCGAACATGTTTTAGCCATT
GTGCGCACCCGACTTTCTGCCGCTGGTGAAGATCCCGCGCACATGACCGATGGCGAGT
TGTTAGAAAAAGCTCCCAGCTACTTTGCTGGTGGTCATGACGTCATCATCGGACGCCG
ACACTTTGTCGATGACGCAACCACCAGTGATCAATTGGCCTCATCTGGAACACTGACC
 
GTTAAAGAGCATGAGGCGTTCATCCGCCTGACTGTCGATGGCATCAGGGATTTGTACCA
CCGCAACCGTTACGCACCGTATGTAGTGGCGTTTCAAAACTGGTTGAAACCCGCCGGC
GCGTCTTTTGACCATCTTCATAAACAGCTCGTCGCCATTGATGAACGCGGCCGACTTAT
TGCCGATGAACTGCATCATCTACGTGGCAATCCCAATATGTACAACGAACTTGCTGTTG
ATTACGCCGGATACCACAACCTGATCATCGCGGAAAACGATCACGCCGTGGCCTTCGC
AGGTTTCGGTCACCGCTACCCCACCATTGAGATTTACTCTAAGTCCGCTATTCCTGAAC
CCTGGCTTCAAAGCGACGAGGAAATCCAAGCGATGAGCAACCTCATCCATGCATGCCA
TGCTGCAACCGGCGCAGATGTACCCTGCAATGAGGAATGGGTACACAAACCAATCGAT
GTTGATATGCCAATGCCCTGGCATGTGATGATCAAATGGCGTGTTTCTACCCTGGCAGG
TTTTGAAGGTGGCACCAAGGTGTATCTCAATACGCTGTCTCCACACAAGGTCCGAGAC
CGTGTGGTGAAAGAAATGTACCGACTACGCGATGAAGAACTCATCGCATCTGATCTGC
GCATCGCCATGTAATGCTCCGTTGAACGCAACAGCCTTAAATACAATCCCCTCCTATAA
**SEQ ID No. 6: protein sequence (i.e., amino acid sequence 428aa encoded by Sequence 5) of gene mutant NCgl2002^{E414*}** 
MADGTIKQIHPFTGTEVWTVPGRGNRPLSHPASTIVELSAHDHTSYCAFCSDNMLSTPPEK
SRIIIDSSGDFDILPGALPGELSETTPEFRRVPNLFEIVSFDYWHQNFGFDMDSETAMRMAQ
YLAIPEGREHVLAIVRTRLSAAGEDPAHMTDGELLEKAPSYFAGGHDVIIGRRHFVDDATT
SDQLASSGTLTVKEHEAFIRLTVDGIRDLYHRNRYAPYVVAFQNWLKPAGASFDHLHKQL
VAIDERGRLIADELHHLRGNPNMYNELAVDYAGYHNLIIAENDHAVAFAGFGHRYPTIEIY
SKSAIPEPWLQSDEEIQAMSNLIHACHAATGADVPCNEEWVHKPIDVDMPMPWHVMIKW
RVSTLAGFEGGTKVYLNTLSPHKVRDRVVKEMYRLRDEELIASDLRIAM*CSVERNSLKY
NPLL
**SEQ ID No. 7: Up-NCgl2002E414*-Down sequence (nucleotide sequence of 1022 bp) of point mutation-integrated homologous arm obtained by overlap PCR through P2/P5** 
CAGTGCCAAGCTTGCATGCCTGCAGGTCGACTCTAGGAACTGCATCATCTACGTGGCA
ATCCCAATATGTACAACGAACTTGCTGTTGATTACGCCGGATACCACAACCTGATCATC
GCGGAAAACGATCACGCCGTGGCCTTCGCAGGTTTCGGTCACCGCTACCCCACCATTG
AGATTTACTCTAAGTCCGCTATTCCTGAACCCTGGCTTCAAAGCGACGAGGAAATCCA
AGCGATGAGCAACCTCATCCATGCATGCCATGCTGCAACCGGCGCAGATGTACCCTGC
AATGAGGAATGGGTACACAAACCAATCGATGTTGATATGCCAATGCCCTGGCATGTGAT
GATCAAATGGCGTGTTTCTACCCTGGCAGGTTTTGAAGGTGGCACCAAGGTGTATCTC
AATACGCTGTCTCCACACAAGGTCCGAGACCGTGTGGTGAAAGAAATGTACCGACTAC
GCGATGAAGAACTCATCGCATCTGATCTGCGCATCGCCATGTAATGCTCCGTTGAACGC
AACAGCCTTAAATACAATCCCCTCCTATAAGCCAAGAGTTTTAGTGTCGCTGCGCAGGT
ACTCTACTATCTAATCCATGAGCCGCATTTCAGAACTTCTAAACAATCATGGTGTTGATC
 
TGTCGTGGCAAGAGGCCGCATATCAGGATTTCCACGAACATCCTGAGCTCTCCGGCTTC
GAATCAGAGACCGCAGATCGCATTCAGAAATACCTCGAGCGTTTTGATTGTGAGGTGA
TTCCAAATGTTGGCGGTTACGGCATTCTGGCCGTGTTCCGAAATGGGTCGACAGATCCT
GGTGCCCCTGTTGCGTTAATGCGCGCAGATTTCGATGGCCTTCCCGTCAAGGAAATCAC
CGGAGTTCCGTTTGCTTCCACTCGTATGCGTCCGCATGATGGGGCAAATGTCCATGTCA
TGCACGCATGCGGCCACGATGTCCACGTCACCGCGCTGCTTGGTGCGGGTACCGAGCT
CGAATTCGTAATCATGGTCATAGCTG
**SEQ ID No. 8: DNA sequence (nucleotide sequence of 1097 bp) of homologous arm, from which NCgl2002 was knocked out, as obtained through P8/P11**

CAGTGCCAAGCTTGCATGCCTGCAGGTCGACTCTAGCGTGATGCAGGCCGAAGGATCG
CTCAAGAATACGTAGCTCAAAACTAGCGAAGGATCTCCACAGTCCATGGGTAGACTGC
AGGAACTTCATAGGCAAGTTCCTGCTCCAGGAGGAAAGCGTCCTTTGGCAACTCTGCT
GGCGGGGTGAGCAAACGGGCAAAAATCATGGTCACTTGGTTAACGGAGCCTTTAACTC
CATCAACTTCAATGAGGTAACGGTTGACCGAGGATTCTTCCAAGTCTGCATCTTCATGC
TCGTCGCGGTAAGTCTGGCGAAGCTGCTCTTCCACAGCTTCTGCGATTTTCGCTGCTGG
ATCATGGACAACAACGCCATGATCCATGAGGCCTTTTTCGATCAAAAGGTTGGCGACTA
CGGTAAAACGTCCCGCTAGTGCGATGGAATCTGTGTCGGGGACTAAAACATCAAACTG
AATCTTCGGCATAACACCACAGTAGACAATAGCCTTGGTGTTATGACTAGCCCCCATTC
TTTTTCTGTCACCCCCATTCGCACCGCCAAGAGTTTTAGTGTCGCTGCGCAGGTACTCT
ACTATCTAATCCATGAGCCGCATTTCAGAACTTCTAAACAATCATGGTGTTGATCTGTCG
TGGCAAGAGGCCGCATATCAGGATTTCCACGAACATCCTGAGCTCTCCGGCTTCGAATC
AGAGACCGCAGATCGCATTCAGAAATACCTCGAGCGTTTTGATTGTGAGGTGATTCCA
AATGTTGGCGGTTACGGCATTCTGGCCGTGTTCCGAAATGGGTCGACAGATCCTGGTG
CCCCTGTTGCGTTAATGCGCGCAGATTTCGATGGCCTTCCCGTCAAGGAAATCACCGGA
GTTCCGTTTGCTTCCACTCGTATGCGTCCGCATGATGGGGCAAATGTCCATGTCATGCA
CGCATGCGGCCACGATGTCCACGTCACCGCGCTGCTTGGTGCGTGTGCCATTTTAGATG
AGCGTCGCGATGCATGGGAAGGCACGTTCATCGCGTTGTTCCAGCCATCGGAGGAAAA
CTCCGGGTACCGAGCTCGAATTCGTAATCATGGTCATAGCTG

### Industrial applications

Experiments have shown that mutating or knocking out the coding gene of the protein as shown in SEQ ID No. 2 can increase the yield of L-lysine in cells. L-lysine can be prepared by mutating or knocking out the coding gene of the protein as shown in SEQ ID No. 2 in cells. The present invention has good application prospects.

## Claims

1. Use of a substance for knocking out a protein coding gene or inhibiting the protein content or activity in the preparation of L-lysine;
the protein comprisesA1) or A2) as follows:
A1) a protein having an amino acid sequence of SEQ ID No. 2; and
A2) a fusion protein obtained by linking a tag to an N-terminal or/and a C-terminal of A1).

2. The use according to claim 1, wherein the coding gene comprises b1) or b2) or b3) as follows:
b1) a DNA molecule as shown in SEQ ID No. 1 in a sequence listing;
b2) a DNA molecule that has 75% or more identity to a nucleotide sequence defined by b1) and encodes the protein; and
b3) a DNA molecule that hybridizes with the nucleotide sequence defined by b1) or b2) under stringent conditions and encodes the protein.

3. The use according to claim 1 or 2, **characterized in that**, the substance comprises B1) or B2):
B1) a nucleic acid molecule that reduces the expression level of the protein according to claim 1; and
B2) an expression cassette, a recombinant vector, a recombinant microorganism or a transgenic cell line containing the nucleic acid molecule of B1).

4. The use according to any one of claims 1 to 3, **characterized in that**, the substance is a substance that mutates a glutamate residue codon at position 414 of SEQ ID No. 2 to a terminator; or
the substance is a substance that mutates a guanine nucleotide at position 1240 of SEQ ID No. 1 into a thymine nucleotide.

5. A method for preparing L-lysine, comprising: reducing the content or activity of the protein of claim 1 in a recipient biological cell, or knocking out an coding gene of the protein of claim 1 in the recipient biological cell to obtain a recombinant biological cell; and culturing the recombinant biological cell to obtain the L-lysine.

6. The method according to claim 5, **characterized in that**, the biological cell is a yeast, a bacterium, algae, a fungus, a plant cell or an animal cell that is capable of synthesizing the L-lysine.

7. The method according to claim 6, wherein the bacterium is Corynebacterium glutamicum.

8. The method according to any one of claims 5 to 7, **characterized in that**, the recombinant biological cell is cultured using a medium that enables the recombinant biological cell to grow; and/or
the recombinant biological cell is cultured using conditions that enable the recombinant biological cell to grow.

9. The method according to any one of claims 5 to 7, **characterized in that**, the method is achieved by mutating a glutamate residue codon at position 414 of SEQ ID No. 2 in the recipient biological cell into a terminator, or mutating a guanine nucleotide at position 1240 of SEQ ID No. 1 into a thymine nucleotide, or knocking out the gene as shown in SEQ ID No. 1.

10. The method according to claim 9, **characterized in that**, the recombinant biological cell is cultured using a medium that enables the recombinant biological cell to grow; and/or
the recombinant biological cell is cultured using conditions that enable the recombinant biological cell to grow.

11. A biological material which comprises b1) or b2) or b3) or b4) or b5) as follows:
b1) a DNA molecule that encodes a protein having an amino acid sequence as shown in SEQ ID No. 6;
b2) a DNA molecule that has 75% or more identity to the sequence of the DNA molecule defined by b1) and encodes a protein as shown in SEQ ID No. 6;
b3) a DNA molecule that hybridizes with a nucleotide sequence defined by b1) or b2) under stringent conditions and encodes a protein as shown in SEQ ID No. 6;
b4) an expression cassette, a recombinant vector, a recombinant microorganism or a transgenic cell line containing the DNA molecule of b1) or b2) or b3); and
b5) the recombinant biological cell of any one of claims 5 to 9.

12. A product for preparing L-lysine, which contains the substance of any one of claims 1 to 4 or the biomaterial of claim 11.
